# EUROPEAN PATENT SPECIFICATION

(11) **EP 4 142 808 B1**
(45) Date of publication and mention of the grant of the patent: **17.07.2024**
(21) Application number: 21722839.4
(22) Date of filing: 29.04.2021
(51) Int. Cl.: A61L 2/00, A61L 2/14, A61L 2/22

(54) **STERILISATION APPARATUS FOR PRODUCING PLASMA AND HYDROXYL RADICALS**
STERILISATIONSVORRICHTUNG ZUR ERZEUGUNG VON PLASMA- UND HYDROXYLRADIKALEN
APPAREIL DE STÉRILISATION POUR LA PRODUCTION DE PLASMA ET DE RADICAUX HYDROXYLE

(30) Priority: 30.04.2020 GB 202006383
(43) Date of publication of application: 08.03.2023
(73) Proprietor: Creo Medical Limited, Chepstow, Monmouthshire NP16 5UH (GB)
(72) Inventor: HANCOCK, Christopher Paul, Chepstow, Monmouthshire NP16 5UH (GB); ULLRICH, George, Bethesda, Gwynedd LS57 4YY (GB); WEBB, David Edward, Bethesda Gwynedd LL57 4YY (GB); TURNER, Louis, Chepstow, Monmouthshire NP16 5UH (GB); HODGKINS, George, Chepstow, Monmouthshire NP16 5UH (GB); PRESTON, Shaun, Chepstow, Monmouthshire NP16 5UH (GB); MEADOWCROFT, Simon, Chepstow, Monmouthshire NP16 5UH (GB)
(74) Representative: Mewburn Ellis LLP
(86) International application number: PCT/EP2021/061331
(87) International publication number: WO 2021/219820

(56) References cited:
- WO-A1-2019/175063
- WO-A1-2019/211276
- US-A1- 2010 021 340

## Description

### FIELD OF THE INVENTION

The invention relates to sterilisation systems suitable for clinical use, e.g. on the human body, medical apparatuses, or hospital bed spaces. For example, the invention may provide a system that can be used to destroy or treat certain bacteria and/or viruses associated with the human or animal biological system and/or the surrounding environment. This invention is particularly useful for sterilising or decontaminating enclosed or partially enclosed spaces.

### BACKGROUND TO THE INVENTION

Bacteria are single-celled organisms that are found almost everywhere, exist in large numbers and are capable of dividing and multiplying rapidly. Most bacteria are harmless, but there are three harmful groups; namely: cocci, spirilla, and bacilla. The cocci bacteria are round cells, the spirilla bacteria are coil-shaped cells, and the bacilli bacteria are rod-shaped. The harmful bacteria cause diseases such as tetanus and typhoid.

Viruses can only live and multiply by taking over other cells, i.e. they cannot survive on their own. Viruses cause diseases such as colds, flu, mumps and AIDS. Viruses may be transferred through person-to-person contact, or through contact with region that is contaminated with respiratory droplets or other virus-carrying bodily fluids from an infected person.

Fungal spores and tiny organisms called protozoa can cause illness.

Sterilisation is an act or process that destroys or eliminates all form of life, especially micro-organisms. During the process of plasma sterilisation, active agents are produced. These active agents are high intensity ultraviolet photons and free radicals, which are atoms or assemblies of atoms with chemically unpaired electrons. An attractive feature of plasma sterilisation is that it is possible to achieve sterilisation at relatively low temperatures, such as body temperature. Plasma sterilisation also has the benefit that it is safe to the operator and the patient.

Plasma typically contains charged electrons and ions as well as chemically active species, such as ozone, nitrous oxides, and hydroxyl radicals. Hydroxyl radicals are far more effective at oxidizing pollutants in the air than ozone and are several times more germicidal and fungicidal than chlorine, which makes them a very interesting candidate for destroying bacteria or viruses and for performing effective decontamination of objects contained within enclosed spaces, e.g. objects or items associated with a hospital environment.

OH radicals held within a "macromolecule" of water (e.g. a droplet within a mist or fog) are stable for several seconds and they are 1000 times more effective than conventional disinfectants at comparable concentrations.

An article by Bai et al titled "Experimental studies on elimination of microbial contamination by hydroxyl radicals produced by strong ionisation discharge" (Plasma Science and Technology, vol. 10, no. 4, August 2008) considers the use of OH radicals produced by strong ionisation discharges to eliminate microbial contamination. In this study, the sterilisation effect on E. coli and B. subtilis is considered. The bacteria suspension with a concentration of 10⁷ cfu/ml (cfu = colony forming unit) was prepared and a micropipette was used to transfer 10 µl of the bacteria in fluid form onto 12 mm x 12 mm sterile stainless steel plates . The bacteria fluid was spread evenly on the plates and allowed to dry for 90 minutes. The plates were then put into a sterile glass dish and OH radicals with a constant concentration were sprayed onto the plates. The outcomes from this experimental study were:
1. OH radicals can be used to cause irreversible damage to cells and ultimately kill them;
2. The threshold potential for eliminating micro-organisms is ten thousandths of the disinfectants used at home or abroad;
3. The biochemical reaction with OH is a free radical reaction and the biochemical reaction time for eliminating micro-organisms is about 1 second, which meets the need for rapid elimination of microbial contamination, and the lethal time is about one thousandth of that for current domestic and international disinfectants;
4. The lethal density of OH is about one thousandths of the spray density for other disinfectants - this will be helpful for eliminating microbial contamination efficiently and rapidly in large spaces, e.g. bed-space areas; and
5. The OH mist or fog drops oxidize the bacteria into CO₂, H₂O and micro-inorganic salts. The remaining OH will also decompose into H₂O and O₂, thus this method will eliminate microbial contamination without pollution.

WO 2009/060214 discloses sterilisation apparatus arranged controllably to generate and emit hydroxyl radicals. The apparatus includes an applicator which receives RF or microwave energy, gas and water mist in a hydroxyl radical generating region. The impedance at the hydroxyl radical generating region is controlled to be high to promote creation of an ionisation discharge which in turn generates hydroxyl radicals when water mist is present. The applicator may be a coaxial assembly or waveguide. A dynamic tuning mechanism e.g. integrated in the applicator may control the impedance at the hydroxyl radical generating region. The delivery means for the mist, gas and/or energy can be integrated with each other.

WO 2019/175063 A1 discloses a sterilization apparatus which uses thermal or non-thermal plasma to disinfect surgical scoping devices such as endoscopes, gastroscopes, laparoscopes and the like.

### SUMMARY OF THE INVENTION

At its most general, the invention provides a sterilisation device arranged to generate and deflect a thermal or non-thermal plasma into a flow of water mist in order to provide a stream containing hydroxyl radicals. The stream may be directed on to a surface or object to perform sterilisation.

According to the invention, there is provided a sterilisation device for generating a flow of hydroxyl radicals, the sterilisation device comprising: a housing defining a water mist fluid flow path between an inlet for receiving water mist and an outlet for directing a flow of hydroxyl radicals towards a region to be sterilised; an energy delivery tip configured to produce a thermal or non-thermal plasma; a coaxial transmission line mounted in the housing and arranged to convey radiofrequency (RF) and/or microwave frequency electromagnetic (EM) energy to the energy delivery tip; and a gas conduit mounted in the housing and arranged to deliver gas to the energy delivery tip, wherein the coaxial transmission line comprises an inner conductor, an outer conductor, and a dielectric material separating the inner conductor from the outer conductor, wherein the energy delivery tip extends from a distal end of the coaxial transmission line, and wherein the energy delivery tip comprises: a first electrode electrically connected to the inner conductor of the coaxial transmission line; and a second electrode electrically connected to the outer conductor of the coaxial transmission line, wherein the second electrode is configured to define an internal volume of the energy delivery tip, wherein the first electrode extends longitudinally within the internal volume, wherein the first electrode and second electrode are configured to: strike a plasma in the gas delivered to the energy delivery tip, and direct the plasma into the water mist fluid flow path to form hydroxyl radicals therein.

In use, the sterilisation device is configured to produce a plasma, which is directed into a flow path of water mist to produce hydroxyl radicals for sterilisation. The device may also be capable of use in the absence of the water mist, where sterilisation is performed by the plasma alone.

The plasma may be directed by the first electrode and second electrode in a longitudinal direction parallel with an axis of the coaxial transmission line. The water mist flow path may be intersect the plasma as it exits the energy delivery tip. Alternatively, the plasm may be deflected by the first electrode and second electrode in a transverse direction that intersects a longitudinally directed water mist fluid flow path. These arrangement facilitate a longitudinally directed water mist fluid flow path, which is useful for providing a directable sterilisation beam.

The device may be a handheld unit. For example, the housing may be a portable unit, e.g. comprising a handle or grip. Providing a handheld unit may facilitate directing the flow of hydroxyl radicals to sterilise any surface or object as required.

The energy delivery tip advantageously defines a bipolar (e.g. coaxial) structure to produce a high electric field from received RF and/or microwave frequency energy in the internal volume to strike and sustain a thermal or non-thermal plasma in the gas present in the volume. For example, a short pulse (e.g. having a duration of 10 ms or less, e.g. between 1 ms and 10 ms) of RF energy may be used to strike the plasma. A longer microwave pulse may be used to sustain the plasma.

However, it may also be possible to strike the plasma using the microwave frequency energy, e.g. by using a microwave resonator or an impedance transformer, i.e. a quarter wave transformer that transforms a low voltage to a higher voltage to strike plasma using a higher impedance transmission line that is a quarter wave (or an odd multiple thereof) long at the frequency of operation. This high impedance line may be switched in to strike plasma and switched out (i.e. to return to a lower impedance line) once the plasma has been struck and it is required to sustain plasma. A power PIN or varactor diode may be preferably used to switch between the two states, although it may be possible to use a co-axial or waveguide switch.

Alternatively, the energy delivery tip may be configured to strike and sustain plasma using only microwave energy through the provision of two quarter wavelength impedance transformed at the distal end of the coaxial transmission line. A first quarter wavelength transformer is configured to reduce the impedance of the coaxial transmission line, e.g. from 50 Q to a target impedance (e.g. 25 Q or the like). Once struck, the plasma in the internal volume will present a lower impedance, and hence the transformation can assist with power delivery into the struck plasma in order to sustain it.

A second (distalmost) quarter wavelength transformer is then provided with a much higher impedance, e.g. 200 Q or more. The second quarter wavelength transformer operates to transform the lower impedance at the distal end of the first transformer to a much higher distal impedance, e.g. of 1600 Q. Assuming a lossless line and an input microwave power of 100 W, a voltage of 400 V is achievable at the distal end of the second quarter wavelength transformer. The dimensions of the first and quarter wavelength transformers can be configured to provide a distal voltage capable of striking a plasma.

Preferably, the energy delivery tip is located within the housing. The first electrode and second electrode may define an exit from the internal volume, wherein the exit is located at or in the outlet. The first electrode may be an elongate element extending in the longitudinal direction. It may be straight, or may be provided as another shape. For example, in some embodiments the first electrode may be helical. Optionally, the first electrode may be formed of a portion of the inner conductor that extends beyond a distal end of the outer conductor.

The energy delivery tip may be open at its distal end to form the opening for directing plasma out of the energy delivery tip. In such embodiments, the plasma is directed out of the energy delivery tip in a longitudinal direction. Alternatively, the energy delivery tip may comprise a conductive cap mounted on the first electrode at a distal end of the internal volume, the conductive cap being spaced from a distal end of the second electrode to define the outlet for directing plasma out of the internal volume and into the fluid flow path, wherein plasma is directed radially outwards of the energy delivery tip at into the fluid flow path. For example, the fluid flow path may be formed coaxially with the energy delivery tip. The conductive cap may ensure that plasma is efficiently produced and helps to direct substantially all of the plasma into the fluid flow path to maximised production of hydroxyl radicals. The conductive cap effectively acts as an extension of the first electrode for generation or plasma.

Advantageously, the fluid flow path tapers towards the outlet. In this way, the velocity of hydroxyl radicals may be increased, such that the radicals are more effectively projected towards a surface or object which is to be sterilised. In some embodiments, particularly where the energy delivery tip is located at or about the outlet, tapering of the flow path in this way ensures that substantially all of the water mist passes through a plasma to maximise the production of hydroxyl radicals.

Preferably, the energy delivery tip further comprises an insulating cap mounted at a distal end of the coaxial transmission line to isolate the coaxial transmission line from the internal volume, and wherein the gas conduit is in fluid communication with the internal volume via a flow path formed between the insulating cap and the second electrode. The insulating cap may be mounted within the second electrode, e.g. to define a proximal end of the internal volume. The flow path may comprise a plurality of openings in the second electrode that permit gas flow around the insulating cap. The plurality of openings may be regularly space to facilitate a uniform flow of gas into the internal volume.

The insulating cap may help to ensure that plasma is generated in a distal part of the energy delivery tip, and may also help to direct generated plasma out of the energy delivery tip. In some embodiments, the insulating cap may have a chamfered distal end in the region of an opening through the second electrode. This may help to increase velocity of gas along the flow path the second electrode, aiding throughput of gas and direction of plasma out of the distal end of the energy delivery tip.

The second electrode may be a cylinder. The plurality of openings may each comprise a longitudinal notch in the cylinder. For example, a proximal end of the second electrode may be castellated to provide the plurality of openings.

The energy delivery tip may comprise an insulating dielectric material located between the first electrode and the second electrode in the internal volume. The dielectric material may be a piece or quartz, or other similar low loss material. Preferably the dielectric material is provided as a cylinder to sit within the second electrode. The dielectric material causes an increase in the electric field in the gas-filled gap beside the insulation dielectric material which aids the production of plasma. In addition, the dielectric material reduces the size of the internal volume of the energy delivery tip, which increases the flow rate of gas therethrough and so plasma is projected further from the energy delivery tip. This may be particularly advantageous in embodiments where plasma is directed out of the tip longitudinally, and/or where the device is configured to sterilisation using plasma only.

According to a second aspect of the invention there is provided a sterilisation apparatus comprising a sterilisation device according to the first aspect, a mist generator, a gas supply, and a generator for supplying radiofrequency (RF) and/or microwave frequency electromagnetic (EM) energy to the handheld sterilisation device. RF EM energy may be for striking the plasma, and may be received as a high voltage pulse. The microwave EM energy may be for sustaining the plasma, i.e. delivering power into the plasma to maintain the state of ionisation. This may also be received as a pulse. The plasma may be struck repeatedly in a manner to produce a quasi-continuous beam of plasma.

The mist generator may comprise either an ultrasonic transducer or a heating element. In this way, the mist generator may supply a mist (e.g. moisture or fog) to the handheld sterilisation device for the production of hydroxyl radicals from water. As a water mist is provided, the apparatus does not need to use any chemical cleaning agents, and so no harmful by-products result from sterilisation using the present apparatus.

Preferably the gas supply is a supply of argon gas. However, any other suitable gas may be chosen, e.g. carbon dioxide, helium, nitrogen, a mixture of air and any one of these gases, for example 10% air/90% helium.

Advantageously, the generator may be powered by a battery, such that the generator is portable. Preferably the mist generator and the gas supply are also portable such that a user may easily operate the sterilisation apparatus, and sterilisation can be easily performed in any necessary environment.

Herein, the term "inner" means radially closer to the centre (e.g. axis) of the coaxial transmission line, energy delivery tip, and/or applicator. The term "outer" means radially further from the centre (axis) of the coaxial transmission line, energy delivery tip, and/or applicator.

The term "conductive" is used here to mean electrically conductive, unless the context dictates otherwise.

Herein, the terms "proximal" and "distal" refers to the ends of the applicator. In use, the proximal end is closer to a generator for providing the RF and/or microwave energy, whereas the distal end is further from the generator.

In this specification "microwave" may be used broadly to indicated a frequency range of 400 MHz to 100 GHz, but preferably in the range 1 GHz to 60 GHz. Specific frequencies that have been considered are: 915 MHz, 2.45 GHz, 3.3 GHz, 5.8 GHz, 10 GHz, 14.5 GHz, and 25 GHz. In contrast, this specification uses "radiofrequency" or "RF" to indicate a frequency range that is at least three orders of magnitude lower, e.g. up to 300 MHz, preferably 10 kHz to 1MHz, and most preferably 400 kHz. The microwave frequency may be adjusted to enable the microwave energy delivered to be optimised. For example, an energy delivery tip may be designed to operate at a certain frequency (e.g. 900 MHz), but in use the most efficient frequency may be different (e.g. 866 MHz).

### BRIEF DESCRIPTION OF THE DRAWINGS

Features of the invention are now explained in the detailed description of examples of the invention given below with reference to the accompanying drawings, in which:
Fig. 1 is a schematic diagram of a sterilisation apparatus according to an embodiment of the present invention;
Fig. 2 is a diagram of an applicator;
Fig. 3 is a cross-sectional view of the applicator shown in Fig. 2; and
Fig. 4 is a cross-sectional view of a second applicator.

### DETAILED DESCRIPTION; FURTHER OPTIONS AND PREFERENCES

This invention relates to a device for performing sterilisation using hydroxyl radicals that are generated by creating a plasma in the presence of water mist.

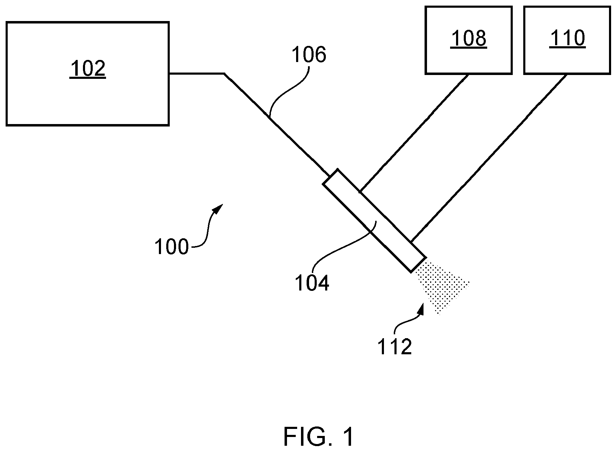

Fig. 1 is a schematic diagram of a sterilisation apparatus 100 which is an embodiment of the present invention. The apparatus 100 is capable of generating hydroxyl (OH) radicals in order to sterilise a surface or an area. For example, the apparatus 100 may be used to sterilise medical apparatuses or hospital bed spaces.

The apparatus 100 comprises a generator 102 which able to controllably deliver radiofrequency (RF) and/or microwave energy to an applicator 104. The generator may be of type disclosed in WO 2012/076844, for example. The generator 102 is connected to the applicator 104 by a coaxial cable 106. The coaxial cable 106 comprises an inner conductor, an outer conductor and a dielectric material separating the inner conductor from the outer conductor. The coaxial cable 106 may couple energy into the applicator 104 through a QMA connector or the like. In some examples, the generator 102 may be arranged to monitor reflected signals (i.e. reflected power) received back from the applicator 104 in order to determine an appropriate signal to be conveyed to the applicator 104. The radiofrequency and/or microwave energy is utilised at the applicator 104 in order to strike and sustain a thermal or non-thermal plasma in order to generate hydroxyl radicals in a manner which is explained in more detail below. In some examples, the thermal or non-thermal plasma may be used directly to sterilise surfaces.

The apparatus 100 further comprises a mist generator 108, which is arranged to deliver water mist (e.g. moisture or fog) to the applicator 104. The mist generator 108 may generate a mist by means of an ultrasonic transducer, for example. Alternatively, the mist generator 108 may be arranged to heat water to generate steam or mist to be passed to the applicator 104. The mist is supplied to the applicator 104 in order to generate hydroxyl radicals by a process which will be explained in more detail below. By using mist generator 108 in this way the apparatus 100 can be used to sterilise surfaces or objects without the use of any cleaning chemicals, reducing costs associated with sterilisation and allowing sterilisation to be performed when cleaning chemicals are in short supply. The use of hydroxyl radicals for sterilisation also ensures that there are no harmful by-products.

The mist generator 108 may include a pump or other fluid driving unit to cause generated mist to flow towards the applicator 104.

A gas supply 110 is connected to the applicator 104 to supply gas for forming a plasma which is used to generate hydroxyl radicals in a manner which will be explained below. The gas supply 110 may be a supply of any suitably inert gas for formation of a non-thermal or thermal plasma, for example argon, helium, nitrogen, carbon dioxide or a combination thereof. The gas supply 110 may be configured to allow adjustment of the flow rate of gas which is delivered to the applicator 104. The gas supply can supply between 1.5 and 10 litres of gas per minute, for example.

It may not be essential to provide an independent driving unit for mist flow. Instead, the mist may naturally diffuse towards the applicator. The mist may be entrained by a flow of gas, which in turn will accelerate the diffusion rate of the mist.

The flow rate of gas may be in the range 1.5 to 15 litres/minute, preferably between 2 and 6 litres/minute. The mist generator 108 may be arranged to generate sufficient mist for the mist to form at least 2% by volume of the combined gas/mist stream. The flow rate of the gas may be controlled to reach a desired proportion of gas and mist in the combined stream.

In some embodiments of the invention it is envisaged that the generator 102, the mist generator 108 and the gas supply 110 may each be portable, and the applicator 104 may be a handheld applicator such that the present invention provides an effective sterilisation apparatus which is easily transportable by a user.

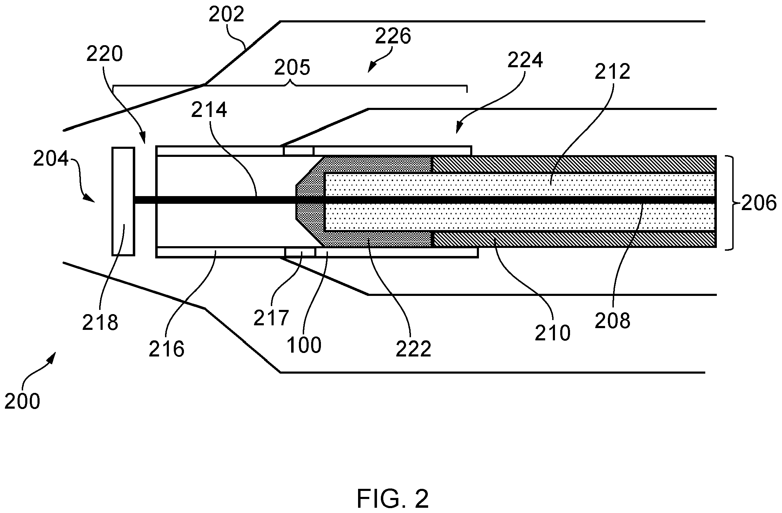

The applicator 104 is shown in more detail in Figs. 2 to 4 below. To sterilise a surface, a plasma is created in the applicator 104 by applying energy from the generator 102 to the gas delivered from the gas supply 110. For example, RF energy may be used to strike a plasma and microwave energy may be used to sustain the plasma. For example, plasma may be generated as disclosed in WO 2009/060213. Simultaneous with the generation of plasma, water mist from the mist generator 108 is passed through a housing of the applicator 104 along a fluid flow path which passes through the plasma in order to produce a spray 112 of hydroxyl radicals which pass out of the applicator 104 to be directed at a surface or into an area for sterilisation. Examples of hydroxyl radical generation in this manner are disclosed in WO 2009/060214, for example.

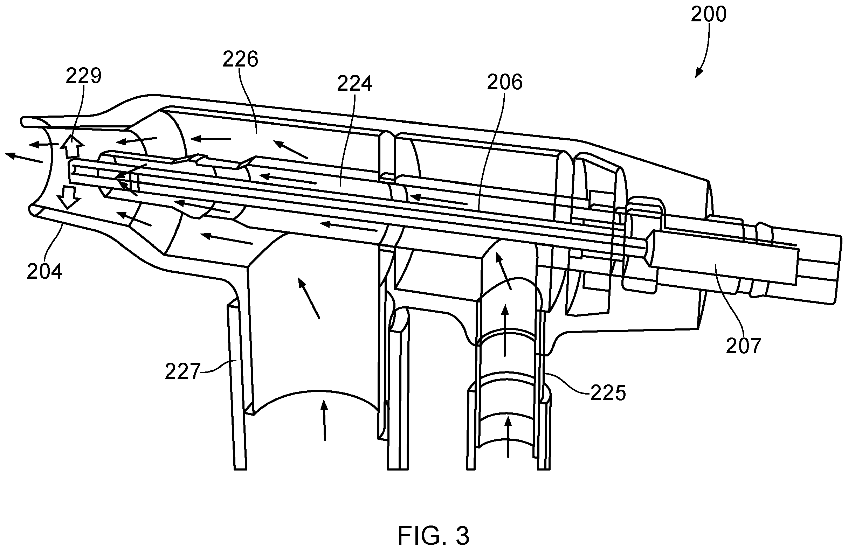

Fig. 2 shows a schematic view of a first applicator 200 which may be used in an embodiment of the present invention. In particular, Fig. 2 shows detail regarding an energy delivery tip of the applicator 200. A cross-sectional view showing the applicator more generally is shown in Fig. 3.

The applicator 200 may be produced at any suitable scale. For example, the applicator may be sized to be gripped by a human hand. Alternatively, a larger version suitable for mounted on a stand may be manufactured. In use, the stream of plasma and/or OH radicals emitted by the applicator may be directed into a volume to be sterilized, e.g. the inside of a vehicle (e.g. ambulance) or a hospital bed or surgical suite.

The applicator 200 comprises a generally elongate housing 202, which contains components required to generate hydroxyl radicals which are directed out of a distal outlet 204 towards a surface or object to be sterilised. In particularly preferred embodiments the applicator 200 may be handheld by a user to manually pass the applicator 200 over the surface or object.

Within the housing 202, the applicator 200 comprises an energy delivery structure that comprises a coaxial transmission line 206 that has an energy delivery tip 205 at a distal end thereof. A proximal end of coaxial transmission line 206 terminates in a QMA connector 207 or the like provided such that RF and/or microwave energy may be introduced to the energy delivery structure from a generator, e.g. via a cable as discussed above in relation to Fig. 1.

The coaxial transmission line 206 comprises an inner conductor 208, an outer conductor 210 and a dielectric material 212 separating the inner conductor 208 and the outer conductor 210. The coaxial transmission line 206 lies along a longitudinal axis of the housing 202.

The energy delivery tip 205 comprises a first electrode 214 electrically connected to the inner conductor 208, a second electrode 216 electrically connected to the outer conductor 210, and a conductive end cap 218, which is spaced away from the distal end of the second electrode 216 to define a gap 220. In this embodiment, the second electrode 216 is provided as a hollow cylinder which is open at each end, and the first electrode 214 is positioned generally along the longitudinal axis of the second electrode 216. An annular space is thereby defined between the first electrode 214 and the second electrode 216.

The first electrode 214 may be formed by an extension of the inner conductor 208 of the coaxial transmission line 206. The first electrode 214 is preferably straight, though in some examples the first electrode 214 may be provided in other shapes. For example, the first electrode 214 may be a helical electrode.

The conductive end cap 218 is a disc of conductive material (e.g. copper, silver, gold or plated steel) that is electrically connected to the first electrode 214 and shaped to lie over the mouth of the opening to the second electrode 216. The conductive end cap 218 is displaced from the distal end of the second electrode 216 in the longitudinal direction to form the gap 220. The gap 220 is smaller than the radial separation of the first electrode 214 and second electrode 216, i.e. the radial distance between the outer surface of the first electrode 214 and the inner surface of the hollow cylinder that forms the second electrode 216. For example, the gap 220 between the end cap 218 and the second electrode 216 may have a length in the longitudinal direction of around 0.5 mm.

The first electrode 214, second electrode 216 and conductive end cap 218 are configured such that, when energy (e.g. an RF signal) is supplied to the coaxial transmission line 206, a high voltage condition is set up in and around the gap 220. The input signal may be control to cause the voltage to be high enough at the gap 220 to strike a plasma in gas flowing through or past an outer surface of the probe tip 205.

The effect of the geometry of the end cap 218 is to create a plasma generation region within the probe tip 205 at its distal end. Thermal or non-thermal plasma may be struck and sustain in this region through suitable delivery of RF and microwave energy through the coaxial transmission line 206 as is known.

As discussed below, in this embodiment the gas from which the plasma is formed is supplied to the annular space formed between the first electrode 214 and the second electrode 216. The gas flows through the probe tip 205 in the longitudinal direction. At the plasma generation region, the gas or plasma is diverted by the end cap 218 in a radial direction to intersect with a flow path 226 of water mist, as described below.

Within the second electrode 216, positioned at the distal end of the coaxial transmission line 206, is a generally cylindrical ceramic cap 222, which may extend for around 2 mm in the distal direction beyond the end of the dielectric material 212. In some embodiments, the first electrode 214 may be connected to the inner conductor 208 of the coaxial transmission line 206 by a conductive element which extends through the ceramic cap 222.

A gas conduit 224 is formed around the coaxial transmission line 206 in order to deliver gas to the energy delivery tip. At a proximal end of the applicator 200 the gas conduit 224 comprises a connector 225 to receive gas from the gas supply 110. Gas is able to flow from the gas conduit 224 to within the second electrode 216 through castellations or apertures 217 which are formed in the proximal end of the second electrode 216. It may be desirable to have a plurality of castellations or apertures spaced regularly around the circumference of the second electrode 216 so that the flow of gas into the energy delivery tip if substantially uniform around the longitudinal axis.

Around the gas conduit 224, a fluid flow path 226 is formed in the applicator housing 202 to direct water mist from an inlet 227 to the outlet 204. As shown in Fig. 2, the fluid flow path 226 generally tapers in a distal direction towards the outlet 204. This serves the dual function of bringing the water mist into contact with the plasma being emitted from the gap 220 and to increase the flow rate of the mist. The plasma causes generation of hydroxyl radicals in the water mist, which are then ejected through the outlet 204 as a directable flow or spray.

In use, the gas supply 110 is operated to pass gas into the applicator 200 and through the gas conduit 224. The ceramic cap 222 has a chamfered distal face to encourage gas flowing from the gas conduit 224 into the second electrode 216 to pass between the first electrode 214 and the second electrode 216, where a thermal or non-thermal plasma is struck. For example, a plasma may be struck using RF energy, and sustained using microwave energy. Of course, it is envisaged that in some embodiments either RF or microwave energy may be used to strike and sustain the plasma. The power supplied to sustain the plasma may be controlled to maintain the plasma in a preferred state, e.g. as a non-thermal plasma.

At the same time as the plasma is produced, the mist generator 108 passes gas into the applicator 200 to provide a flow of water mist along the fluid flow path 226. The plasma passes out of the second electrode 216 through the gap 220, which forms a circumferential outlet for directing plasma into the fluid flow path 226 of water mist passing through the applicator 200. The plasma is shown in Fig. 3 by arrows 229. As the water mist passes through the plasma, the water molecules are ionised to produce hydroxyl radicals which pass through the outlet 204 and towards a surface or object to be sterilised.

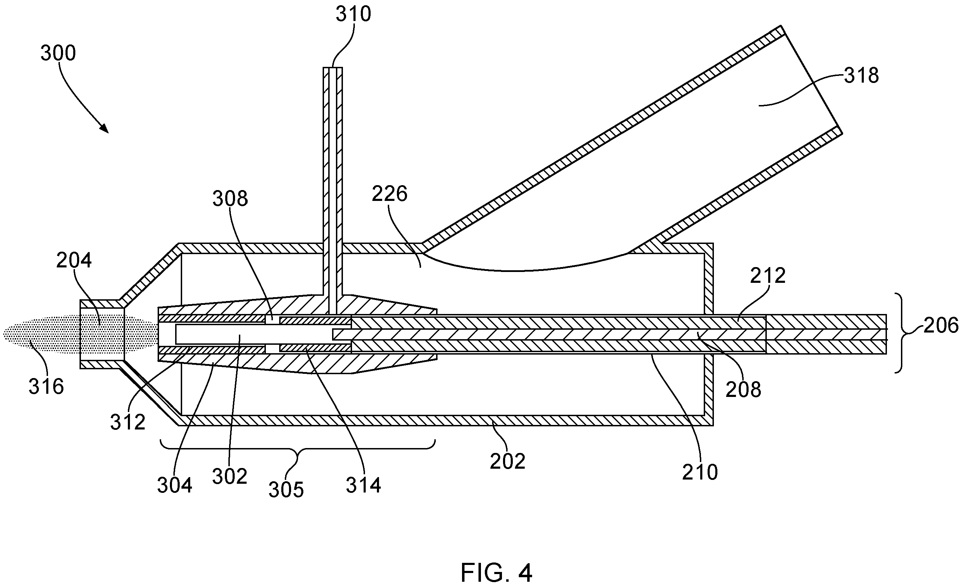

Fig. 4 shows a cross-sectional view of a second applicator 300 which may be used in an embodiment of the invention. In particular, the applicator 300 is adapted such that it may be used to sterilise surfaces or objects using hydroxyl radicals or using a thermal or non-thermal plasma which is emitted from the applicator 300. Features of the second applicator 300 which correspond with the first applicator 200 have been given the same reference numerals, and are not described again.

The applicator 300 comprises an energy delivery tip 305 connected to the distal end of a coaxial transmission line 206. The energy delivery tip 305 is configured to produce a thermal or non-thermal plasma which passes through the outlet 204 of the applicator 300. In this way, the applicator 300 may be used for sterilisation using just plasma when a gas supply, such as gas supply 110, is activated, or may be operable to produce hydroxyl radicals when the gas supply and a mist generator, such as mist generator 108, are both activated.

The energy delivery tip 305 comprises a first electrode 302 connected to the inner conductor 208 of the coaxial transmission line 206. In some embodiments, the first electrode 302 may be a continuation of the inner conductor 208. A second electrode 304 is connected to the outer conductor 210 of the coaxial transmission line 206. In this embodiment, the second electrode 304 is provided as a hollow cylinder which is open at each end, and the first electrode 302 is positioned generally along the longitudinal axis of the second electrode 304. In the embodiment shown, the first electrode 302 has a length of 20 mm, and the distal end of the first electrode 302 is 2 mm from the distal end of the second electrode 304. The spacing between the first electrode 302 and the second electrode 304 in the radial direction is approximately 1.3 mm. The first electrode 302 and the second electrode 304 are arranged to define an annular region 308 therebetween for receiving gas from a gas inlet 310, wherein a gas conduit is formed from the gas inlet, through the second electrode 304 and into the annular region 308 between the first electrode 302 and the second electrode 304.

At a distal end of the energy delivery tip 305 a quartz tube 312 is positioned within the annular region 308, with a gap between the quartz tube 312 and the first electrode 302 through which gas is able to flow. For example, the quartz tube 312 may have a length of 12.35 mm, and may be positioned such that the distal end of the quartz tube 312 is coterminous with the distal end of the second electrode 304. By positioning the quartz tube 312 in this way, the electric field in the gas-filled gap between the first electrode 302 and the second electrode 304 is increased to facilitate a plasma strike. The region within the quartz tube 312 is thus a plasma generation region.

At a proximal end of the energy delivery tip, an insulating element is provided to separate the coaxial transmission line 206 and the plasma generation region. The insulating element in this example comprises a ceramic cap 314 which sits around a proximal region of the first electrode 302. There is a gap between the cap 314 and the second electrode 304 in order to provide a conduit for gas to flow into the energy delivery tip from the inlet 310. For example, the cap 314 may have a length of 8 mm and an outer diameter of approximately 4.3 mm. The cap 314 is provide to prevent striking of the plasma at the proximal end of the energy delivery tip. In some examples the distal end of the cap 314 may be chamfered to encourage gas to flow between the quartz tube 312 and the first electrode 302, where the plasma is struck.

A distal end of the second electrode 304 is open to form an outlet for plasma generated within the energy delivery tip. In this way, plasma is directed in a longitudinal direction out of the energy delivery tip and towards the outlet 204 of the applicator 300. This is shown in Fig. 4 as a plume 316 of plasma which projects outwardly from the outlet 204 where it may be directed onto a surface or an object by a user for sterilisation.

The applicator 300 may be used in either a first mode for sterilisation using hydroxyl radicals, or a second mode for sterilisation using plasma.

In a first mode, the gas supply 110 is operated to pass gas into the applicator 300 via the inlet 310. The gas passes into the region between the first electrode 302 and the quartz tube 312 where a plasma is struck. For example, a plasma may be struck using RF energy, and sustained using microwave energy. Of course, it is envisaged that in some embodiments either RF or microwave energy may be used to strike and sustain the plasma. At the same time as the plasma is produced, the mist generator 108 passes water mist into the applicator 300 via a mist inlet 318 to provide a flow of water mist along the fluid flow path 226. The plasma passes out of the second electrode 216 in a longitudinal direction, directing plasma into the fluid flow path 226 of water mist passing through the outlet 204. As the water mist passes through the plasma plume 316, the water molecules are ionised to produce hydroxyl radicals which pass through the outlet 204 and towards a surface or object to be sterilised.

The second mode comprises the same steps as the first mode, but a water mist is not supplied to the applicator 300, such that only a thermal or a non-thermal plasma is produced as plume 316 directed out of the outlet 204. The plume 316 of plasma may be passed directly over surfaces by a user for sterilisation.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

## Claims

1. A sterilisation device (200, 300) for generating a flow of hydroxyl radicals, the sterilisation device (200, 300) comprising:
a housing (202) defining a water mist fluid flow path between an inlet (227) for receiving water mist and an outlet (204) for directing a flow of hydroxyl radicals towards a region to be sterilised;
an energy delivery tip (205, 305) configured to produce a thermal or non-thermal plasma;
a coaxial transmission line (206) mounted in the housing (202) and arranged to convey radiofrequency (RF) and/or microwave frequency electromagnetic (EM) energy to the energy delivery tip (205, 305); and
a gas conduit (224) mounted in the housing (202) and arranged to deliver gas to the energy delivery tip (205, 305),
wherein the coaxial transmission line (206) comprises an inner conductor (208), an outer conductor (210), and a dielectric material (212) separating the inner conductor (208) from the outer conductor (210),
wherein the energy delivery tip (205, 305) extends from a distal end of the coaxial transmission line (206), and
wherein the energy delivery tip (205, 305) comprises:
a first electrode (214, 302) electrically connected to the inner conductor (208) of the coaxial transmission line (206); and
a second electrode (216, 304) electrically connected to the outer conductor (210) of the coaxial transmission line (206), wherein the second electrode (216, 304) is configured to define an internal volume of the energy delivery tip, wherein the first electrode (214, 305) extends longitudinally within the internal volume,
wherein the first electrode (214, 302) and second electrode (216, 304) are configured to:
strike a plasma in the gas delivered to the energy delivery tip (205, 305), and
direct the plasma into the water mist fluid flow path to form hydroxyl radicals therein.

2. A sterilisation device (200, 300) according to claim 1, wherein the energy delivery tip (205, 305) is located within the housing (202).

3. A sterilisation device (200, 300) according to claim 2, wherein the first electrode (214, 302) and second electrode (216, 304) define an exit from the internal volume, wherein the exit is located at or in the outlet (204).

4. A sterilisation device (200, 300) according to any preceding claim, wherein the first electrode (214) is formed from a length of the inner conductor (208) that extends beyond a distal end of the outer conductor (210).

5. A sterilisation device (200, 300) according to any preceding claim, wherein the first electrode (214) comprises a conductive end cap (218) mounted beyond a distal end of the second electrode (216), the conductive end cap (214) being configured to deflect the plasma transversely into the water mist fluid flow path.

6. A sterilisation device (200, 300) according to any preceding claim, wherein the water mist fluid flow path tapers towards the outlet (204).

7. A sterilisation device (200, 300) according to any preceding claim, wherein the energy delivery tip (205, 305) further comprises an insulating cap (222, 314) mounted at a distal end of the coaxial transmission line (206) to isolate the coaxial transmission line (206) from the internal volume, and
wherein the gas conduit (224) includes a fluid pathway formed between the insulating cap (222, 314) and the second electrode (216, 304) .

8. A sterilisation device (200, 300) according to claim 7, wherein the insulating cap (222) is mounted within the second electrode, and wherein the fluid pathway comprises a plurality of openings in the second electrode (216, 304) that permit gas flow around the insulating cap (222).

9. A sterilisation device (200, 300) according to claim 8, wherein the second electrode (216, 304) is a cylinder, and the plurality of openings each comprise a notch in the cylinder.

10. A sterilisation device (200, 300) according to claim 9, wherein a proximal end of the second electrode (216, 304) is castellated to provide the plurality of openings.

11. A sterilisation device (200, 300) according to any preceding claim, wherein the energy delivery tip (205, 305) comprises an insulating dielectric material (314) located between the first electrode (214, 302) and the second electrode (216, 304) in the internal volume.

12. A sterilisation device (200, 300) according to claim 11, wherein the dielectric material (314) is quartz.

13. A sterilisation device (200, 300) according to any preceding claim, wherein the housing (202) is configured as a handheld unit.

14. A sterilisation apparatus (100) comprising:
A sterilisation device (104, 200, 300) according to any one of claims 1 to 12;
a mist generator (108);
a gas supply (110); and
a generator (102) for supplying radiofrequency (RF) and/or microwave frequency electromagnetic energy to the sterilisation device (104, 200, 300).

15. A sterilisation apparatus (100) according to claim 14, wherein the mist generator (108) comprises either:
an ultrasonic transducer, or
a heating element.

## Patentansprüche

1. Sterilisationsvorrichtung (200, 300) zur Erzeugung eines Stroms von Hydroxylresten, wobei die Sterilisationsvorrichtung (200, 300) Folgendes umfasst:
ein Gehäuse (202), das einen Wassernebelfluidströmungsweg zwischen einem Einlass (227) zum Empfangen von Wassernebel und einem Auslass (204) zum Lenken eines Stroms von Hydroxylresten zu einer zu sterilisierenden Region hin definiert;
eine Energieabgabespitze (205, 305), die ausgelegt ist, um thermisches oder nichtthermisches Plasma zu erzeugen;
eine Koaxial-Übertragungsleitung (206), die im Gehäuse (202) befestigt und angeordnet ist, um elektromagnetische (EM) Hochfrequenz- (HF-) und/oder Mikrowellenfrequenzenergie zur Energieabgabespitze (205, 305) zu übertragen; und
eine Gasleitung (224), die im Gehäuse (202) befestigt und angeordnet ist, um Gas zur Energieabgabespitze (205, 305) zuzuführen,
wobei die Koaxial-Übertragungsleitung (206) einen inneren Leiter (208), einen äußeren Leiter (210) und ein dielektrisches Material (212), das den inneren Leiter (208) vom äußeren Leiter (210) trennt, umfasst,
wobei sich die Energieabgabespitze (205, 305) von einem distalen Ende der Koaxial-Übertragungsleitung (206) aus erstreckt und
wobei die Energieabgabespitze (205, 305) Folgendes umfasst:
eine erste Elektrode (214, 302), die elektrisch mit dem inneren Leiter (208) der Koaxial-Übertragungsleitung (206) verbunden ist; und
eine zweite Elektrode (216, 304), die elektrisch mit dem äußeren Leiter (210) der Koaxial-Übertragungsleitung (206) verbunden ist, wobei die zweite Elektrode (216, 304) ausgelegt ist, um ein Innenvolumen der Energieabgabespitze zu definieren, wobei sich die erste Elektrode (214, 305) der Länge nach im Innenvolumen erstreckt,
wobei die erste Elektrode (214, 302) und die zweite Elektrode (216, 304) ausgelegt sind, um:
ein Plasma im Gas zu zünden, das zur Energieabgabespitze (205, 305) zugeführt wird, und
das Plasma in den Wassernebelfluidströmungsweg zu lenken, um darin Hydroxylreste zu bilden.

2. Sterilisationsvorrichtung (200, 300) nach Anspruch 1, wobei sich die Energieabgabespitze (205, 305) im Gehäuse (202) befindet.

3. Sterilisationsvorrichtung (200, 300) nach Anspruch 2, wobei die erste Elektrode (214, 302) und die zweite Elektrode (216, 304) einen Ausgang aus dem Innenvolumen definieren, wobei sich der Ausgang am oder im Auslass (204) befindet.

4. Sterilisationsvorrichtung (200, 300) nach einem der vorangegangenen Ansprüche, wobei die erste Elektrode (214) aus einer Länge des inneren Leiters (208) besteht, die sich über ein distales Ende des äußeren Leiters (210) hinaus erstreckt.

5. Sterilisationsvorrichtung (200, 300) nach einem der vorangegangenen Ansprüche, wobei die erste Elektrode (214) eine leitende Endkappe (218) umfasst, die über einem distalen Ende der zweiten Elektrode (216) hinweg befestigt ist, wobei die leitende Endkappe (214) ausgelegt ist, um das Plasma transversal in den Wassernebelfluidströmungsweg abzulenken.

6. Sterilisationsvorrichtung (200, 300) nach einem der vorangegangenen Ansprüche, wobei der Wassernebelfluidströmungsweg zum Auslass (204) hin verjüngt ist.

7. Sterilisationsvorrichtung (200, 300) nach einem der vorangegangenen Ansprüche, wobei die Energieabgabespitze (205, 305) weiters eine Isolierkappe (222, 314) umfasst, die am distalen Ende der Koaxial-Übertragungsleitung (206) befestigt ist, um die Koaxial-Übertragungsleitung (206) vom Innenvolumen zu isolieren, und
wobei die Gasleitung (224) einen Fluidweg umfasst, der zwischen der Isolierkappe (222, 314) und der zweiten Elektrode (216, 304) gebildet ist.

8. Sterilisationsvorrichtung (200, 300) nach Anspruch 7, wobei die Isolierkappe (222) in der zweiten Elektrode befestigt ist und wobei der Fluidweg eine Vielzahl von Öffnungen in der zweiten Elektrode (216, 304) umfasst, die das Strömen von Gas um die Isolierkappe (222) ermöglichen.

9. Sterilisationsvorrichtung (200, 300) nach Anspruch 8, wobei die zweite Elektrode (216, 304) ein Zylinder ist und die Vielzahl von Öffnungen jeweils eine Kerbe im Zylinder umfasst.

10. Sterilisationsvorrichtung (200, 300) nach Anspruch 9, wobei ein proximales Ende der zweiten Elektrode (216, 304) mit Vertiefungen versehen ist, um die Vielzahl von Öffnungen bereitzustellen.

11. Sterilisationsvorrichtung (200, 300) nach einem der vorangegangenen Ansprüche, wobei die Energieabgabespitze (205, 305) ein isolierendes dielektrisches Material (314) umfasst, das sich zwischen der ersten Elektrode (214, 302) und der zweiten Elektrode (216, 304) im Innenvolumen befindet.

12. Sterilisationsvorrichtung (200, 300) nach Anspruch 11, wobei das dielektrische Material (314) Quarz ist.

13. Sterilisationsvorrichtung (200, 300) nach einem der vorangegangenen Ansprüche, wobei das Gehäuse (202) als handgehaltene Einheit ausgelegt ist.

14. Sterilisationsgerät (100), das Folgendes umfasst:
eine Sterilisationsvorrichtung (104, 200, 300) nach einem der Ansprüche 1 bis 12;
einen Nebelerzeuger (108);
eine Gaszufuhr (110); und
einen Generator (102) zum Zuführen von elektromagnetischer Hochfrequenz- (HF-) und/oder Mikrowellenfrequenzenergie zur Sterilisationsvorrichtung (104, 200, 300).

15. Sterilisationsgerät (100) nach Anspruch 14, wobei der Nebelerzeuger (108) entweder
einen Ultraschallwandler oder
ein Heizelement umfasst.

## Revendications

1. Dispositif de stérilisation (200, 300) pour générer un écoulement de radicaux hydroxyles, le dispositif de stérilisation (200, 300) comprenant :
un boîtier (202) définissant un trajet d'écoulement de fluide de brouillard d'eau entre une entrée (227) pour recevoir un brouillard d'eau et une sortie (204) pour diriger un écoulement de radicaux hydroxyles vers une région à stériliser ;
une pointe de distribution d'énergie (205, 305) configurée pour produire un plasma thermique ou non thermique ;
une ligne de transmission coaxiale (206) montée dans le boîtier (202) et agencée pour transporter de l'énergie électromagnétique (EM) radiofréquence (RF) et/ou hyperfréquence vers la pointe de distribution d'énergie (205, 305) ; et
un conduit de gaz (224) monté dans le boîtier (202) et agencé pour distribution du gaz à la pointe de distribution d'énergie (205, 305),
dans lequel une ligne de transmission coaxiale (206) présentant un conducteur interne (208), un conducteur externe (210) et un matériau diélectrique (212) séparant le conducteur interne (208) du conducteur externe (210) ;
dans lequel la pointe de distribution d'énergie (205, 305) s'étend à partir d'une extrémité distale de la ligne de transmission coaxiale (206), et
dans lequel la pointe de distribution d'énergie (205, 305) comprend :
une première électrode (214, 302) connectée électriquement au conducteur interne (208) de la ligne de transmission coaxiale (206) ; et
une seconde électrode (216, 304) connectée électriquement au conducteur externe (210) de la ligne de transmission coaxiale (206), dans lequel la seconde électrode (216, 304) est configurée pour définir un volume interne de la pointe de distribution d'énergie, dans lequel la première électrode (214, 305) s'étend longitudinalement à l'intérieur du volume interne,
dans lequel la première électrode (214, 302) et la seconde électrode (216, 304) sont configurées pour :
amorcer un plasma dans le gaz distribué à la pointe de distribution d'énergie (205, 305), et
diriger le plasma dans le trajet d'écoulement de fluide de brouillard d'eau pour y former des radicaux hydroxyles.

2. Dispositif de stérilisation (200, 300) selon la revendication 1, dans lequel la pointe de distribution d'énergie (205, 305) est située à l'intérieur du boîtier (202)

3. Dispositif de stérilisation (200, 300) selon la revendication 2, dans lequel la première électrode (214, 302) et la seconde électrode (216, 304) définissent une sortie à partir du volume interne, dans lequel la sortie est située au niveau de ou dans la sortie (204).

4. Dispositif de stérilisation (200, 300) selon l'une quelconque des revendications précédentes, dans lequel la première électrode (214) est formée à partir d'une longueur du conducteur interne (208) qui s'étend au-delà d'une extrémité distale du conducteur externe (210).

5. Dispositif de stérilisation (200, 300) selon l'une quelconque des revendications précédentes, dans lequel la première électrode (214) comprend un capuchon d'extrémité conducteur (218) monté au-delà d'une extrémité distale de la seconde électrode (216), le capuchon d'extrémité conducteur (214) étant configuré pour dévier le plasma transversalement dans le trajet d'écoulement de fluide de brouillard d'eau.

6. Dispositif de stérilisation (200, 300) selon l'une quelconque des revendications précédentes, dans lequel le trajet d'écoulement de fluide de brouillard d'eau s'amincit vers la sortie (204).

7. Dispositif de stérilisation (200, 300) selon l'une quelconque des revendications précédentes, dans lequel la pointe de distribution d'énergie (205, 305) comprend en outre un capuchon isolant (222, 314) monté au niveau d'une extrémité distale de la ligne de transmission coaxiale (206) pour isoler la ligne de transmission coaxiale (206) vis-à-vis du volume interne, et
dans lequel le conduit de gaz (224) inclut un trajet de fluide formé entre le capuchon isolant (222, 314) et la seconde électrode (216, 304).

8. Dispositif de stérilisation (200, 300) selon la revendication 7, dans lequel le capuchon isolant (222) est monté à l'intérieur de la seconde électrode, et dans lequel le trajet de fluide comprend une pluralité d'ouvertures dans la seconde électrode (216, 304) qui permettent un écoulement de gaz autour du capuchon isolant (222).

9. Dispositif de stérilisation (200, 300) selon la revendication 8, dans lequel la seconde électrode (216, 304) est un cylindre, et la pluralité d'ouvertures comprennent chacune une encoche dans le cylindre.

10. Dispositif de stérilisation (200, 300) selon la revendication 9, dans lequel une extrémité proximale de la seconde électrode (216, 304) est crénelée pour fournir la pluralité d'ouvertures.

11. Dispositif de stérilisation (200, 300) selon l'une quelconque des revendications précédentes, dans lequel la pointe de distribution d'énergie (205, 305) comprend un matériau diélectrique isolant (314) situé entre la première électrode (214, 302) et la seconde électrode (216, 304) dans le volume interne.

12. Dispositif de stérilisation (200, 300) selon la revendication 11, dans lequel le matériau diélectrique (314) est du quartz.

13. Dispositif de stérilisation (200, 300) selon l'une quelconque des revendications précédentes, dans lequel le boîtier (202) est configuré sous la forme d'une unité portative.

14. Appareil de stérilisation (100), comprenant :
un dispositif de stérilisation (104, 200, 300) selon l'une quelconque des revendications 1 à 12 ;
un générateur de brouillard (108) ;
une alimentation en gaz (110) ; et
un générateur (102) pour fournir de l'énergie électromagnétique radiofréquence (RF) et/ou hyperfréquence au dispositif de stérilisation (104, 200, 300).

15. Appareil de stérilisation (100) selon la revendication 14, dans lequel le générateur de brouillard (108) comprend soit :
un transducteur ultrasonore, soit
un élément chauffant.
